(19) **Europäisches Patentamt**
**European Patent Office**
**Office européen des brevets**

(11) **EP 3 111 987 B1**

(12) **EUROPEAN PATENT SPECIFICATION**

(45) Date of publication and mention
of the grant of the patent:
**20.04.2022 Bulletin 2022/16**

(21) Application number: **15755798.4**

(22) Date of filing: **19.02.2015**

(51) International Patent Classification (IPC):
*A61M 37/00* (2006.01)     *A61K 9/00* (2006.01)

(52) Cooperative Patent Classification (CPC):
**A61K 9/0021; A61M 37/0015;** A61M 2037/0023;
A61M 2037/0053; A61M 2037/0061

(86) International application number:
**PCT/JP2015/054641**

(87) International publication number:
**WO 2015/129545 (03.09.2015 Gazette 2015/35)**

(54) **MICRONEEDLE SHEET**

MIKRONADELBLECH

FEUILLE À MICRO-AIGUILLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **27.02.2014   JP 2014037348**

(43) Date of publication of application:
**04.01.2017   Bulletin 2017/01**

(73) Proprietor: **Hisamitsu Pharmaceutical Co., Inc.
Tosu-shi, Saga 841-0017 (JP)**

(72) Inventors:
• **YAMAMOTO, Naoki
Tsukuba-shi
Ibaraki 305-0856 (JP)**

• **OGURA, Makoto
Tsukuba-shi
Ibaraki 305-0856 (JP)**

(74) Representative: **Grünecker Patent- und
Rechtsanwälte
PartG mbB
Leopoldstraße 4
80802 München (DE)**

(56) References cited:
EP-A1- 1 360 935          WO-A1-2009/051147
WO-A1-2013/187392     WO-A1-2013/187392
WO-A1-2013/191025     WO-A1-2013/191025
JP-A- 2006 345 984       JP-A- 2008 228 958
JP-A- 2010 505 518       JP-A- 2013 153 866
JP-A- 2013 527 853       US-A1- 2011 034 860

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Technical Field**

The present invention relates to a microneedle sheet used for assisting in administration of an active component by microneedles.

**Background Art**

Microneedles for administrating active components through skin and devices including the microneedles are conventionally known. For example, a rotatable microstructure apparatus disclosed in Patent Literature 1 below includes a curved substrate and a roller structure including a plurality of microelements affixed upon a first surface of the substrate. The microelements are of predetermined sizes and shapes so as to penetrate a stratum corneum layer of skin when the microstructure apparatus is placed upon the skin and rolled over the skin in a predetermined direction.

**Citation List**

**Patent Literature**

[Patent Literature 1] Japanese Unexamined Patent Publication No. 2005-503210
WO-A-2013/187392 relates to a microneedle sheet used to assist administration of an active component through microneedles; WO-A-2013/191025 discloses a method for manufacturing a needle-shaped body and a needle-shaped body; EP-A-1 360 935 relates to continuous strips of physiological fluid sampling and testing devices; US-A-2011/0034860 relates to microneedle arrays for delivering a bioactive agent to a therapeutic agent.

**Summary of Invention**

**Technical Problem**

Unfortunately, in the microstructure apparatus disclosed in Patent Literature 1 above, the microelements are exposed on the roller and, therefore, the needles may touch or get caught in other objects (for example, the user's skin or cloth) before an active component is applied on the skin through the microneedles. It is therefore requested to ensure safety during handling of microneedles.

**Solution to Problem**

A microneedle sheet according to the present invention includes a plurality of microneedles formed on a sheet generally along a principal surface of the sheet. The microneedles are formed of a material selected from among polyvinyl alcohol, and a graft copolymer of polyvinyl alcohol and polyethylene glycol. The microneedles are raised from the principal surface by bending the sheet, and the raised microneedles pierce skin. The sheet has a thickness of 30 to 100 $\mu$m. The invention is set out in the appended set of claims.
In the present invention, the microneedles fabricated from a soluble material extend generally along the principal surface of the sheet until the sheet is bent. This means that the tip ends of the microneedles do not protrude from the principal surface before the microneedles are applied to skin. There is therefore no concern that the microneedles touch or get caught in other objects unless the microneedle sheet is applied to skin. As a result, the safety during handling of the microneedles can be ensured. In addition, when compared with a microneedle sheet of a non-soluble material, the microneedle sheet of a soluble material as described above is highly safe, for example, physically less irritating to skin.

**Advantageous** Effects of Invention

According to the present invention, safety during handling of microneedles can be ensured.

Brief Description of Drawings

FIG. 1 is a plan view of a microneedle sheet according to an embodiment.
FIG. 2 is a diagram showing the microneedle sheet fixed to a liner.

FIG. 3 is a perspective view of an exemplary applicator.
FIG. 4 is a diagram showing a usage manner of the applicator shown in FIG. 3.
FIG. 5 is a diagram schematically showing application of the microneedle sheet.
FIG. 6 is a diagram schematically showing application of the microneedle sheet.
FIG. 7 is a diagram schematically showing application of the microneedle sheet.
FIG. 8 is a diagram schematically showing a manner of insertion.
FIG. 9 is a table showing the results of examples.
FIG. 10 is a graph showing the result of a skin permeation test, which is another example.
FIG. 11 is a graph showing the result of the above-noted skin permeation test.

**Description of Embodiments**

Embodiments of the present invention will be described in detail below with reference to the accompanying drawings. In the description of the drawings, the same or equivalent components are denoted with the same reference signs and an overlapping description will be omitted. The methods and fabrication examples mentioned herein do not form part of the invention.

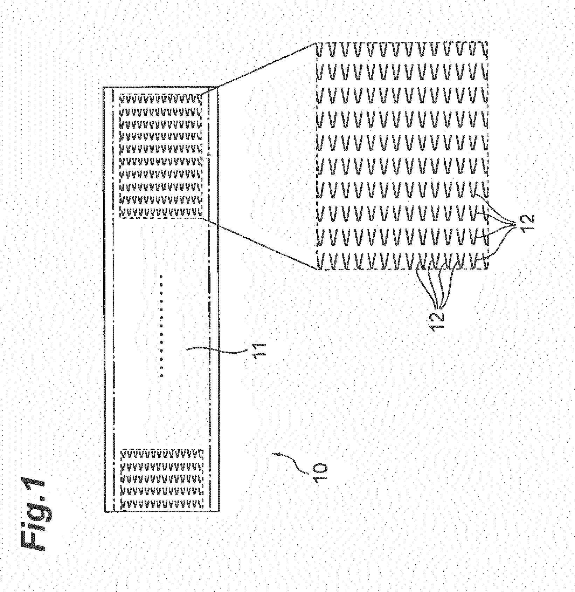

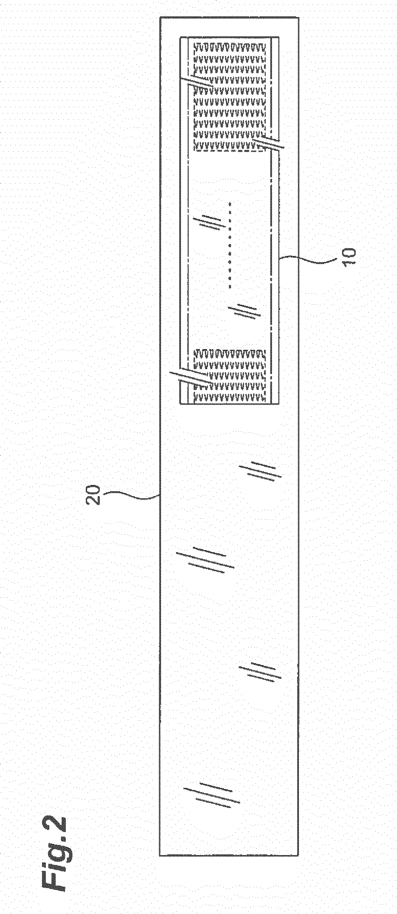

The structure of a microneedle sheet 10 according to an embodiment will be described with reference to FIGs. 1 and 2. The microneedle sheet 10 is an instrument for administering any given active component (for example, drug) into a living body and includes a number of microneedles to pierce skin.

As shown in FIG. 1, the microneedle sheet 10 is shaped like a strip and has a plurality of microneedles 12 formed on a sheet generally along the principal surface 11 of the sheet. These microneedles 12 are arranged in alignment with each of the longitudinal direction and the width direction of the sheet. The tip ends of all of the microneedles 12 are oriented toward one end of the sheet (leftward in FIG. 1) without exception.

The microneedle sheet 10 and the microneedles 12 are of any material. For example, the microneedle sheet 10 and the microneedles 12 may be fabricated from any one of stainless steels, polyethylene terephthalate (PET), other metals, other resins, biodegradable materials, ceramics, and soluble materials. Alternatively, the microneedle sheet 10 and the microneedles 12 may be fabricated from these materials in combination.

The material of the microneedle sheet 10 and the microneedles 12 may be selected from among polyvinyl alcohol (PVA), and polyvinyl alcohol-polyethylene glycol-graft copolymer, and, for example, may be selected from one of these kinds.

As used in the present description, "the material of the microneedle sheet and the microneedles" refers to a substance intentionally used by a producer to fabricate the microneedle sheet and the microneedles. In the production process of the microneedle sheet and the microneedles, a substance that is not selected as a material (for example, a minute amount of impurities) may be unintentionally mixed, or such an unintended substance may not be completely removed. The microneedle sheet and the microneedles according to the present invention encompass the microneedle sheet and the microneedles that eventually include a material not intended by a producer in addition to a substance intended by the producer.

The microneedles 12 can be formed by etching or lasers. When the sheet is metal, the microneedles 12 can be formed by dissolving the sheet with chemicals. When the sheet is non-metal, the microneedles 12 can be formed by evaporating the sheet with a laser. In these cases, a gap is produced around the periphery of the microneedle 12. It is needless to say that the microneedles 12 can be formed by any technique other than lasers and etching. Although the microneedle 12 is shaped like a triangle in the present embodiment as shown in FIG. 1, the microneedle may have any shape. In any case, the microneedle sheet 10 can be produced readily and inexpensively because there is no need for raising the microneedles 12 from the principal surface 11 of the sheet in advance.

The microneedle sheet 10 has a thickness of 10 to 300 $\mu$m and can be fabricated by selecting any one of HPMC, HPC, PVA, and a polyvinyl alcohol-polyethylene glycol-graft copolymer, as a material. The lower limit of the thickness of the microneedle sheet 10 may preferably be 20 $\mu$m, 30 $\mu$m, 40 $\mu$m, or 50 $\mu$m, and the upper limit of the thickness may preferably be 200 $\mu$m, 150 $\mu$m, 100 $\mu$m, 90 $\mu$m, 80 $\mu$m, 70 $\mu$m, 60 $\mu$m, or 50 $\mu$m. The lower limit of the thickness of the microneedle sheet 10 is determined considering the strength of the microneedles 12 inserted into skin, and the upper limit of the thickness is determined considering the flexibility of the sheet, the insertion characteristic of the microneedles 12, and other conditions. The lower limit of the length of the microneedle sheet 10 may be 0.1 cm or 1 cm, and the upper limit of the length may be 50 cm or 20 cm. The lower limit of the width of the microneedle sheet 10 may be 0.1 cm or 1 cm, and the upper limit of the width may be 60 cm or 30 cm. The lower limits of the length and the width of the microneedle sheet 10 are determined considering the dose of active components, and the upper limits of the length and the width are determined considering the size of the living body.

Parameters pertaining to the microneedles 12 may have any value. Specifically, the lower limit of the length of the microneedle 12 may be 10 $\mu$m or 100 $\mu$m, and the upper limit of the length may be 10000 $\mu$m or 1000 $\mu$m. Here, the length of the microneedle 12 is the distance from the bottom of the microneedle 12 (the base portion rising from the principal surface 11) to the apex. The lower limit of the density of needles may be 0.05 needle/cm$^2$ or 1 needle/cm$^2$, and the upper limit of the density may be 10000 needles/cm$^2$ or 5000 needles/cm$^2$. The lower limit of the density is a value obtained in terms of the number of needles and area with which 1 mg of an active component can be administered, and the upper limit of the density is the limit value considering the shape of the needles.

Possible methods of preparing an active component to be applied to skin in the case of the microneedle sheet of a soluble material are: allowing the microneedle sheet 10 per se to contain an active component; coating the microneedle sheet 10 per se with an active component in advance; applying an active component on skin before inserting the microneedles 12 into the skin; and inserting the microneedles 12 into skin and thereafter applying an active component on the skin. If the microneedle sheet 10 is coated with an active component in advance, it is preferable to apply a coating liquid having a predetermined viscosity at a thickness as uniform as possible over the entire sheet. Such application can be easily done because the microneedles 12 extend along the principal surface 11. The coating may be carried out using the principles of screen printing or may be carried out by any other method. If a biodegradable sheet is used, an active component may be contained in the sheet per se.

In the present embodiment, a liner 20 is used for setting the microneedle sheet 10 in an applicator 30 described later. As shown in FIG. 2, this liner 20 is a strip-like sheet having a length and a width larger than those of the microneedle sheet 10. Examples of the material of the liner 20 include plastics such as acrylics, but the material should not be limited thereto and any material, for example, a metal or any other resin may be used for fabricating the liner 20. Although the liner 20 is illustrated as a transparent or translucent product in the related drawings, the liner 20 may be opaque. The microneedle sheet 10 is fixed to one end side of the liner 20 with tape, adhesive, or other means.

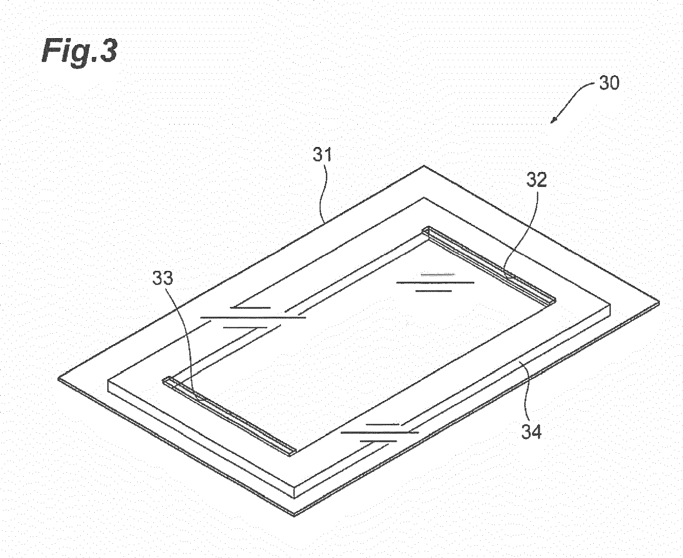

Referring now to FIG. 3, a structure of the applicator 30 will be described. The applicator 30 according to the present embodiment is a rectangular sheet-like instrument used when the microneedle sheet 10 is applied to skin. In the present embodiment, the side provided with an adhesive 34 described later is defined as the lower side of the applicator 30, and the opposite side is defined as the upper side of the applicator 30.

The body 31 of the applicator 30 has two slit-shaped through holes formed in the direction (hereinafter referred to as "width direction") orthogonal to the longitudinal direction. One of the through holes is a hole for guiding the liner 20 and the microneedle sheet 10 from the upper side to the lower side of the body 31 and will hereinafter be referred to as first through hole 32. The other through hole is a hole for guiding the liner 20 stripped from the microneedle sheet 10 from the lower side to the upper side of the body 31 and will hereinafter be referred to as second through hole 33. The distance between the two through holes 32 and 33 may be determined considering the range of application of the microneedle sheet 10 to skin or may be determined considering other criteria.

On the bottom surface of the body 31, an adhesive (adhesive layer) 34 is provided in a rectangular shape so as to surround the two through holes 32 and 33. The adhesive 34 serves the function of fixing the applicator 30 on skin. The adhesive 34 may be provided in any range. For example, the adhesive 34 may be provided only along both edges in the longitudinal direction of the body 31 or may be provided only along both edges in the width direction of the body 31.

Examples of the material of the body 31 include plastics such as acrylics, but the material should not be limited thereto and any material, for example, a metal or any other resin may be used for fabricating the body 31. Although the body 31 is illustrated as a transparent or translucent product in the related drawings, the body 31 may be opaque.

The size of the applicator 30 may be determined in accordance with the size of the microneedle sheet 10 or the liner 20. For example, the width of the applicator 30 may be determined in accordance with the width of the liner 20. The entire length (the length in the longitudinal direction) of the applicator 30 may be determined considering the length of the microneedle sheet 10 or the range of application of the microneedle sheet 10 to skin.

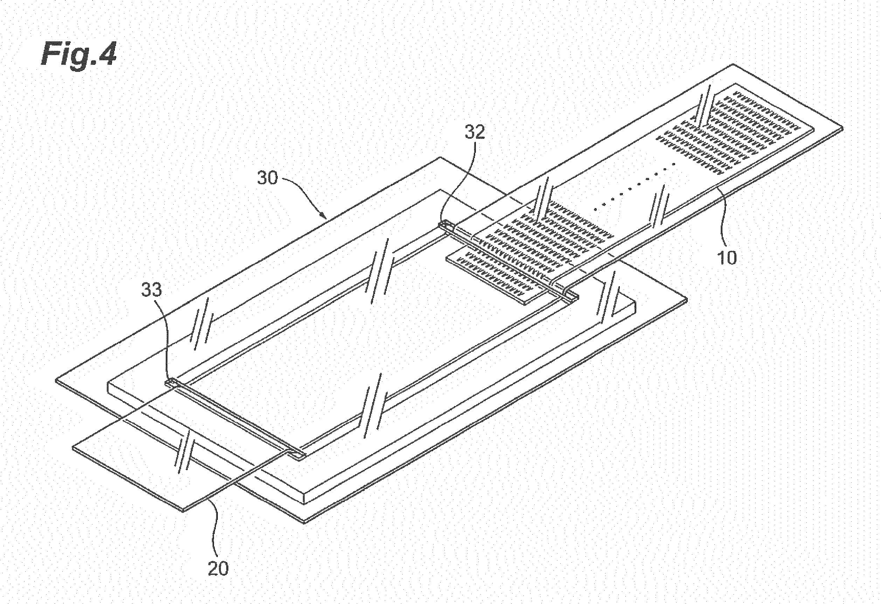

Referring now to FIGs. 4 to 8, the usage manner of the microneedle sheet 10 and the applicator 30 will be described. First, the user sets the liner 20 with the microneedle sheet 10 attached thereto, in the applicator 30. Specifically, the user passes one end of the liner 20 on which the microneedle sheet 10 is not fixed through the first through hole 32 from above to below and further passes the one end through the second through hole 33 from below to above. With this preparation, the liner 20 is positioned on the bottom surface side of the applicator 30 between the two through holes 32 and 33, as shown in FIG. 4.

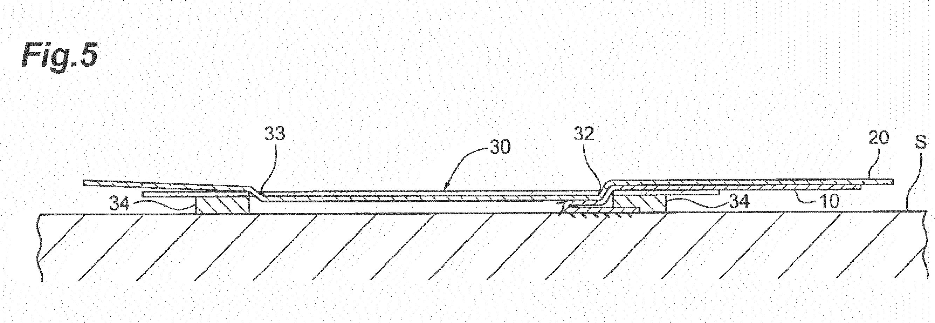

The user then draws one end of the microneedle sheet 10 from the first through hole 32 to the bottom surface side of the applicator 30 and folds the one end such that the one end is positioned below the adhesive 34. Keeping this state, the user affixes the applicator 30 to a site of application of an active component. Through a series of these operations, the applicator 30 is fixed on skin S as shown in FIG. 5.

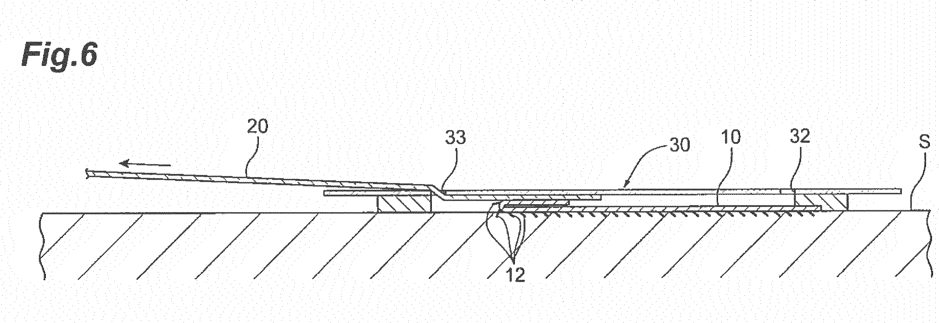

The user then pulls one end of the liner 20 in the direction denoted by the arrow in FIG. 6. Through this operation, the microneedle sheet 10 is guided by its liner 20 to pass through the first through hole 32 and enter the space between the skin S and the bottom surface of the applicator 30.

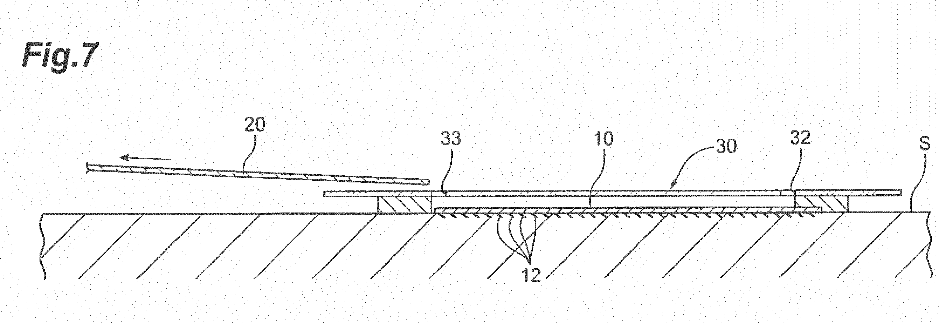

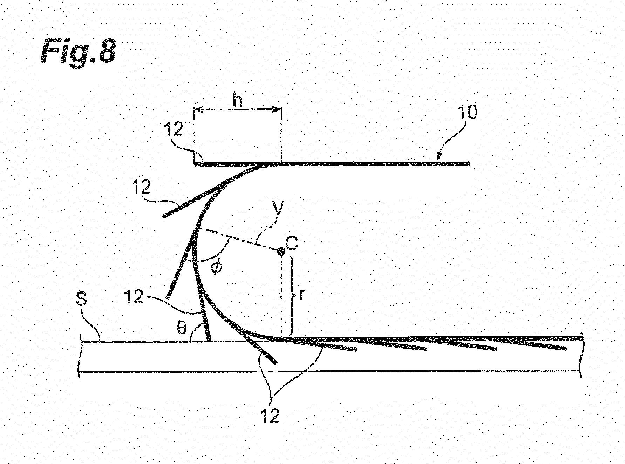

The microneedle sheet 10 is bent by 180 degrees in this space. As shown in FIG. 6 (or as shown in an enlarged view in FIG. 8), the microneedles 12 located at the bent portion are then raised from the principal surface 11, and the raised microneedles 12 pierce the skin S. The user pulls the liner 20 until the entire liner 20 is pulled out of the applicator 30, so that the entire microneedle sheet 10 is applied to the skin as shown in FIG. 7. As is clear from FIGs. 4 to 7, the microneedle sheet 10 (sheet) is moved toward the tip ends of the microneedles 12 and then bent. The microneedles 12 are raised from the principal surface 11 by bending the sheet and then pierce skin.

The user thereafter can peel the applicator 30 from the skin. The user may peel the microneedle sheet 10 immediately or may keep the microneedle sheet 10 applied on the skin S over a predetermined time. When the microneedle sheet 10 is fabricated from a soluble material, it is unnecessary to pull out the microneedles 12 from skin, thereby facilitating the handling of the microneedle sheet 10. Although the microneedle sheet 10 is fixed to the liner 20 with tape or adhesive in the present embodiment, the tape or adhesive can also be used for fixing the microneedle sheet 10 on the skin.

The microneedles 12 forming a row in the width direction of the microneedle sheet 10 are raised at a time between the applicator 30 and the skin S. The angle between the raised microneedle 12 and the principal surface 11 is greater than 0 degrees and less than 180 degrees, as a matter of course.

As shown in FIG. 8, the angle θ of insertion (the angle between the microneedle 12 and the skin S), formed when the microneedle 12 raised from the principal surface 11 pierce skin, is also greater than 0 degrees and less than 180 degrees. The lower limit of the angle of insertion may be 20 degrees, 34 degrees, or 40 degrees, and the upper limit of the angle may be 160 degrees, 140 degrees, or 100 degrees.

The value r in FIG. 8 denotes the radius of curvature of the bent microneedle sheet 10. The maximum angle φ formed between the microneedle 12 raised from the principal surface 11 by folding of the microneedle sheet 10 and an imaginary line V extending from the center of curvature C to the base of the microneedle is greater than 90 degrees. For example, the maximum angle may be in a range of 95 to 130 degrees or may be in a range of 95 to 120 degrees.

The ratio (h/r) of the needle length h to the radius of curvature r is set to be greater than 0.20 to ensure that the microneedle 12 pierces the skin S.

As described above, the microneedle sheet according to an aspect of the present invention includes a plurality of microneedles formed on a sheet generally along the principal surface of the sheet. The material of the microneedles is selected from among hydroxypropyl methylcellulose, hydroxypropyl cellulose, polyvinyl alcohol, and a graft copolymer of polyvinyl alcohol and polyethylene glycol. The microneedles are raised from the principal surface by bending the sheet, and the raised microneedles pierce skin.

In this aspect, the microneedles extend generally along the principal surface of the sheet until the sheet is bent. This means that the tip ends of the microneedles do not protrude from the principal surface before the microneedles are applied to skin. There is therefore no concern that the microneedles touch or get caught in other objects unless the microneedle sheet is applied to skin. As a result, the safety during handling of the microneedles can be ensured. For example, the user can safely carry out storage and conveyance of the microneedle sheet and make preparations immediately before use.

Since the material of the microneedles is a soluble material, it is unnecessary to pull out the microneedles from skin. Furthermore, when compared with a microneedle sheet of a non-soluble material, the microneedle sheet of a soluble material as described above is highly safe, for example, physically less irritating to skin. In addition, the sustained-release drug absorption effect can be expected. The convenience of the microneedle sheet is thus increased.

In the microneedle sheet according to another aspect, the thickness of the sheet may be 10 to 300 μm. Setting the thickness as such makes the microneedle sheet thin and flexible, so that the sheet can be applied on the skin in conformity to the shape of a living body. As a result, an active component can be administered efficiently. By using any one of the four kinds of soluble materials described above, it is possible to fabricate a microneedle sheet that has solubility in a living body and is thinner than ever.

In the microneedle sheet according to another aspect, the angle of insertion of the raised microneedle to skin may be 34 degrees or greater and less than 180 degrees. In this case, the microneedles can be inserted into skin more reliably.

In the microneedle sheet according to another aspect, the maximum angle between the microneedle raised from the principal surface and an imaginary line extending from the center of curvature of the sheet to the base of the microneedle may be greater than 90 degrees. In this case, the length of a part of the microneedle that pierces skin is long to enhance the skin permeation of the active component.

In the microneedle sheet according to another aspect, the maximum angle may be 95 to 130 degrees. In this case, the length of a part of the microneedle that pierces skin is long to enhance the skin permeation of the active component.

Examples

Fabrication of Microneedle sheet

Microneedle sheets were fabricated using a variety of materials. Specifically, each material and water were weighed, and mixed and stirred in a vessel to completely dissolve the material. The material was then defoamed centrifugally at a rotation speed of 2000 rpm to prepare a coating solution. The solution was applied on a liner (mold) so as to attain a thickness after drying of 50 $\mu$m, and dried at 50°C for 1 hour. The polymer films prepared in this manner were subjected to laser processing to produce microneedle sheets. The length of each individual microneedle was 500 $\mu$m, and the density of microneedles was 204 needles/cm$^2$.

The materials used are trehalose, maltose, hyaluronic acid, chondroitin sulfate sodium, dextrin, pullulan, hydroxypropyl methylcellulose (HPMC), hydroxypropyl cellulose (HPC), gelatin, polyacrylic acid, a polyvinyl alcohol-polyethylene glycol-graft copolymer (Kollicoat IR), polyvinyl alcohol (PVA), and two kinds of polyvinyl pyrrolidones (PVP(K30), PVP(K90)). The examples using trehalose, maltose, hyaluronic acid, chondroitin sulfate sodium, dextrin, pullulan, hydroxypropyl methylcellulose (HPMC), hydroxypropyl cellulose (HPC), gelatin, polyacrylic acid, and two kinds of polyvinyl pyrrolidones (PVP(K30), PVP(K90) are reference examples not according to the present invention. The examples using a polyvinyl alcohol-polyethylene glycol-graft copolymer (Kollicoat IR) and polyvinyl alcohol (PVA) are examples in accordance with the present invention.

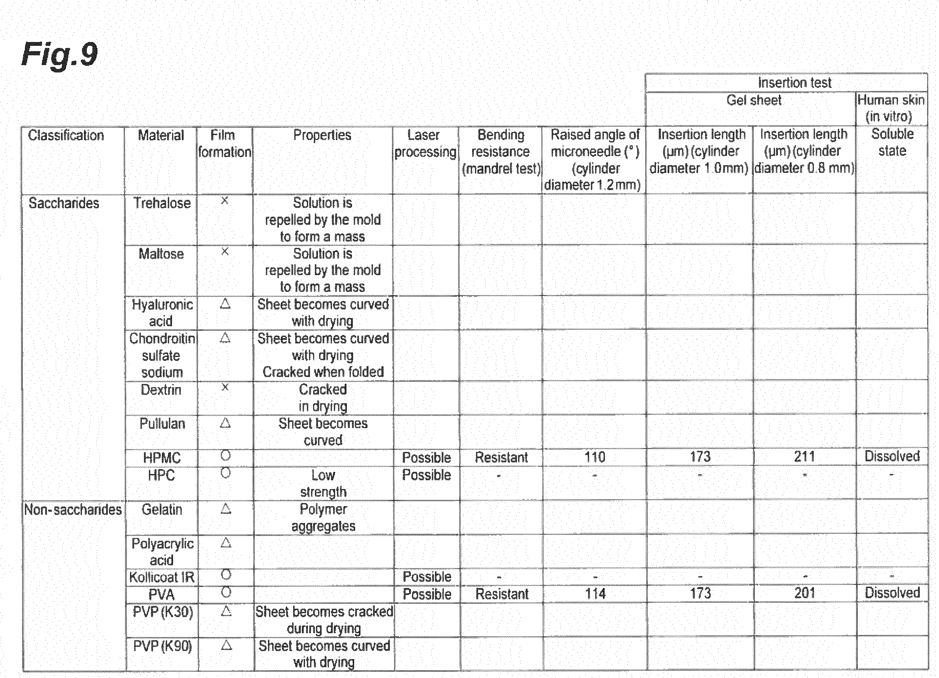

As a result, as shown in the "film formation" and "properties" fields in FIG. 9, when HPMC, HPC, Kollicoat IR, or PVA was used as a material, the microneedle sheet was able to be fabricated satisfactorily. Here, the circle mark in the "film formation" field indicates that a practical microneedle sheet (more specifically, flat microneedle sheet) was able to be fabricated. The triangle mark indicates that a sheet was able to be fabricated but determined to be unpractical because it was curved or any other reasons. The cross mark indicates that a sheet failed to be produced, for example, because the solution was repelled by the mold to form a mass.

Mandrel Test (Bending Resistance Test)

Next, a mandrel test was conducted to evaluate bending resistance for two kinds of microneedle sheets including HPMC and PVA as materials, among four kinds of microneedle sheets that were fabricated satisfactorily. IMC-A0F0 Type of IMOTO MACHINERY CO., LTD. was employed as a bending tester. Specifically, the mandrel (diameter of 1 mm) of the bending tester was set on the center of the microneedle sheet, and the microneedle sheet was folded together with the tester. The folded microneedle sheet was then observed with a microscope to evaluate whether fracture, cracks, or rupture exists.

As a result, as shown in the "bending resistance" field in FIG. 9, it was found that none of fracture, cracks, and rupture occurred in the microneedle sheets fabricated with HPMC and PVA.

Measurement of Raised Angle and Insertion Test Using Gel Sheet

An insertion test was conducted for two kinds of microneedle sheets including HPMC and PVA as materials. A gel sheet was used as a model of skin. Specifically, a rod shaped like a cylinder (diameter of 0.8 to 1.2 mm, hereinafter referred to as "cylindrical rod") was arranged in the width direction of the microneedle sheet, and the microneedle sheet was folded with the cylindrical rod to raise the needles. The cylindrical rod was moved along the upper surface of the gel sheet to cause the microneedles to pierce the gel sheet. In this test, the raised angle and the insertion length were observed. Here, the raised angle is the maximum value of the angle $\phi$ shown in FIG. 8.

In this example, a cylindrical rod having a diameter of 1.2 mm was used in measurement of the raised angle, and cylindrical rods having diameters of 1.0 mm and 0.8 mm were used in measurement of the insertion length. Here, the insertion length is an average value of the lengths of the microneedles intruding into the gel sheet. The insertion length of each individual microneedle was obtained by subtracting the length of the exposed portion from the entire length of the microneedle.

As a result, as shown in FIG. 9, it was found that the microneedle was raised at an angle exceeding 90° in both of the microneedle sheets of HPMC and PVA. It was also found that a length of about 170 to 210 $\mu$m was inserted in the gel sheet at that time. In addition, in the raising and the insertion, rupture of the microneedle sheets was not found.

In Vitro Skin Insertion Test

The solubility of the microneedles was evaluated using human skin for two kinds of microneedle sheets including HPMC and PVA as materials. First, the microneedle sheet was inserted to human skin using a cylindrical rod having a diameter of 1 mm. Then, the microneedle sheet was covered with a polytetrafluoroethylene film, and the microneedle sheet was applied to human skin under a condition of 32°C for 1 hour. Subsequently, the microneedle sheet stripped from the human skin was observed with a microscope.

As a result, it was observed that part of the microneedles and part of the substrate were dissolved when compared with before insertion. That is, it was confirmed that the two kinds of microneedle sheets above also have solubility.

In Vitro Skin Permeation Test

A permeation test of lidocaine hydrochloride to human skin was conducted using a microneedle sheet made of HPMC or PVA, containing lidocaine hydrochloride, to evaluate the amount of the drug administered.

For this test, two microneedle sheets including HPMC as a material and two microneedle sheets including PVA as a material were fabricated. Specifically, lidocaine hydrochloride, the material (HPMC or PVA), and water were weighed, and mixed and stirred in a vessel to completely dissolve the drug and the material. The aqueous solution was then centrifugally defoamed to prepare a coating solution. The solution was applied on a liner (mold) so as to attain a thickness after drying of 50 $\mu$m, and heated for drying. The polymer films prepared in this manner were subjected to laser processing to produce microneedle sheets. Both of the two microneedle sheets including HPMC as a material contained about 25.8% by mass of lidocaine hydrochloride in the finished state. Both of the two microneedle sheets including PVA as a material contained about 8.6% by mass of lidocaine hydrochloride in the finished state. The area of each microneedle sheet was set to 1.5 cm$^2$. The length of each individual microneedle was 500 $\mu$m, and the density of microneedles was about 200 needles/cm$^2$.

A skin permeation test was conducted using a Franz diffusion cell for each of the fabricated four microneedle sheets. Specifically, after the microneedle sheet was applied on the stratum corneum of human skin piece having an area of 5 cm$^2$, the skin piece was attached to the Franz diffusion cell with the dermis facing the receptor chamber, and the cell was kept at 32°C.

Here, "application of the microneedle sheet (to the skin piece)" in this test means encompassing two methods of application. One of the methods is to place the microneedle sheet on the skin piece without inserting individual microneedles into the skin piece, and the other method is to insert individual microneedles into the skin piece. For one of the two microneedle sheets made of HPMC, the microneedles were not inserted into the skin piece, whereas for the other sheet, the microneedles were inserted into the skin piece. Similarly, the microneedles were not inserted into the skin piece for one of the two microneedle sheets made of PVA, whereas the microneedles were inserted into the skin piece for the other.

After attaching the skin piece to the cell, the receptor chamber of this cell was purged with phosphate buffered saline at a certain rate, and the solution in the receptor chamber was sampled at predetermined time intervals. The concentration of lidocaine hydrochloride in each sampled solution was measured by high performance liquid chromatography. The measurement was conducted until 24 hours elapsed since the start. The amount of drug permeation at each time was obtained from the resulting measurement value, and in addition, the skin permeation rate at each time ($\mu$g/cm$^2$/h) and the accumulated amount of permeation ($\mu$g/cm$^2$) were obtained. In addition, the two kinds of use ratios (%) were obtained. The first use ratio (%) is the ratio of the accumulated amount of permeation after the elapse of 24 hours to the amount of drug (lidocaine hydrochloride) initially included in the microneedle sheet (hereinafter referred to as "initial amount"). In addition, the second use ratio (%) is obtained by the equations below using a value called remainder ratio.

$$\text{The second use ratio (\%)} = 100 - \text{the remainder ratio}$$

$$\text{The remainder ratio (\%)} = \{(\text{the amount of drug left in the}$$
$$\text{microneedle sheet after the elapse of 24 hours}) + (\text{the amount of drug}$$
$$\text{adhering to the surface of skin piece})\}/(\text{initial amount}) \times 100$$

Since in the second use ratio, the drug left in the skin piece without reaching to the receptor chamber is also counted into the use ratio, the second use ratio tends to be higher than the first use ratio. The two kinds of use ratios were obtained only in the case where the microneedles were inserted into the skin piece.

The test results are shown in Table 1 below. The "material" field indicates the substance used as a material of the microneedle sheet. The "application" field indicates whether the microneedles were inserted into the skin piece. The maximum skin permeation rate (Jmax) is the maximum value of a plurality of skin permeation rates in each of the microneedle sheets.

[Table 1]

| Material | Application | Drug concentration (%) | Maximum skin permeation rate ($\mu$g/cm²/h) | Accumulated amount of permeation ($\mu$g/cm²) | First use ratio (%) | Second Use ratio (%) |
|---|---|---|---|---|---|---|
| HPMC | Not inserted | 25.8 | 1.1 | 16.4 | | |
| HPMC | Inserted | | 60.9 | 1081.0 | 55.7 | 65.8 |
| PVA | Not inserted | 8.6 | 4.0 | 84.9 | | |
| PVA | Inserted | | 16.9 | 349.4 | 48.8 | 64.9 |

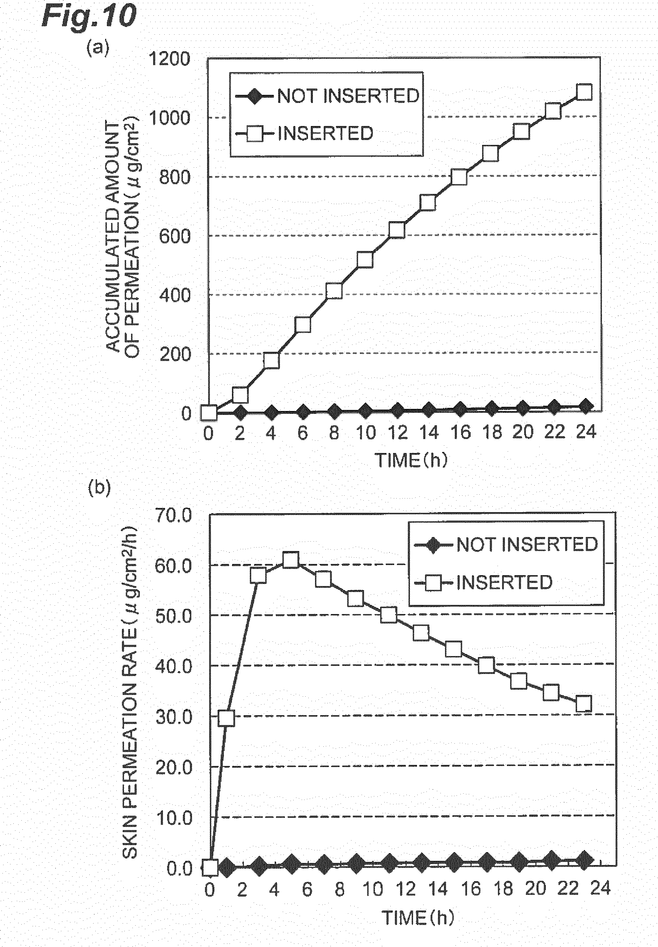

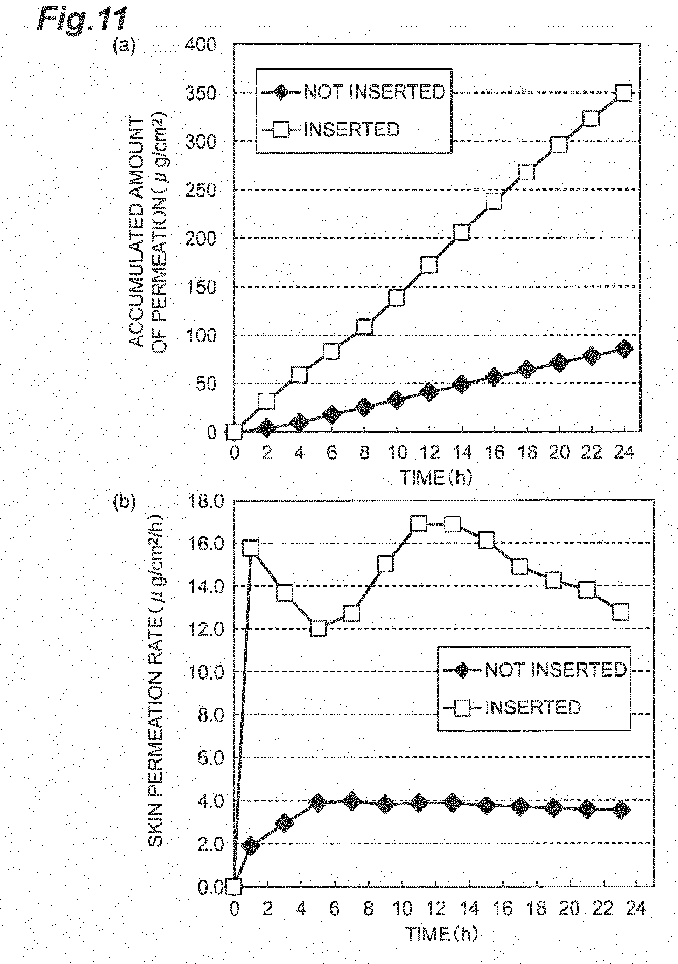

FIG. 10 shows a graph (a) of the accumulated amount of permeation and a graph (b) of the skin permeation rate for the microneedle sheets made of HPMC. FIG. 11 shows a graph (a) of the accumulated amount of permeation and a graph (b) of the skin permeation rate for the microneedle sheets made of PVA. In these two figures, the ordinates and the abscissas in the graph (a) represent the accumulated amount of permeation and the elapsed time (h) since the start of measuring, respectively. The ordinates and the abscissas in the graph (b) represent the skin permeation rate and the elapsed time (h) since the start of measuring, respectively.

These tables and graphs suggest that inserting the microneedles into skin enables the drug to be administered into the body efficiently and also achieves the sustained-release drug absorption effect.

Skin Primary Irritation Test Using Rabbit

A microneedle sheet made of HPMC and a microneedle sheet made of PVA were prepared in accordance with the method explained in the section, Fabrication of Microneedle sheet above. In addition, a microneedle sheet made of stainless steel (SUS) was prepared as a comparative example, in which the length and the density of microneedles were the same as in the microneedle sheets made of HPMC and PVA.

For each of these three kinds of microneedle sheets, skin reaction was evaluated in accordance with the Draize process. Specifically, the microneedle sheet was applied to the shaved back of a rabbit, and the microneedle sheet was fixed to the skin using adhesive tape. In doing so, individual microneedles were inserted into the skin. The microneedle sheet was removed 24 hours after the application, observed for erythema and edema formation with naked eyes, 2 hours, 24 hours, and 48 hours after the removal, and rated based on the Draize (Table 2) evaluation criteria. In a series of these observations, six rabbits were used for each kind of microneedle sheet. Skin primary irritation was assessed by calculating a primary irritation index (P.I.I.) and using the Draize evaluation criteria below (Table 3). The primary irritation index is calculated as follows. First of all, the average score of the six samples was obtained for each of erythema and edema formation 2 hours, 24 hours, and 48 hours after removal of the microneedle sheet. Next, the sum of the average scores was obtained in three groups (2 hours, 24 hours, and 48 hours after removal of the microneedle sheet), and the average of the sums was obtained as a primary irritation index.

[Table 2]

Skin reaction evaluation criteria (Draize)

| Erythema and crust formation | Score |
|---|---|
| No erythema | 0 |
| Very slight erythema (barely perceptible) | 1 |
| Clear erythema | 2 |
| Moderate and severe erythema | 3 |
| Severe erythema (beet red) to slight crust formation (deep damage) | 4 |

| Edema formation | Score |
|---|---|
| No edema | 0 |
| Very slight edema (barely perceptible) | 1 |
| Slight edema (obvious swell that defines clear edge) | 2 |
| Moderate edema (about 1-mm swell) | 3 |
| Severe edema (about 1-mm swell and expansion beyond exposed range) | 4 |

[Table 3]

| Skin primary irritation evaluation criteria (Draize) | |
|---|---|
| 0 = P.I.I. | No irritant |
| 0 < P.I.I. < 2 | Mild irritant |
| 2 ≤ P.I.I. < 5 | Moderate irritant |
| 5 ≤ P.I.I. | Strong irritant |

The evaluation results are shown in Table 4 below. The "material" field indicates the substance used as a material of the microneedle sheet. The symbol "#" in the table indicates that petechiae or petechiae marks were found, the symbol "+" indicates that erythema at the edge or internal hemorrhage was found, and the symbol "$" indicates that squama was found. The bottom row in the score field indicates the sum of the average scores in each group.

[Table 4]

| Materi al | Average value of scores | | | | | | P.I.I | Evaluati on |
|---|---|---|---|---|---|---|---|---|
| | 2 hours | | 24 hours | | 48 hours | | | |
| | Erythe ma | Edem a | Erythe ma | Edem a | Erythe ma | Edem a | | |
| SUS | 1.83#+ | 0.33 | 1.17#+ | 0.00 | 0.83$ | 0.00 | 1.3 9 | Mild |
| | 2.17 | | 1.17 | | 0.83 | | | |
| HPMC | 0.33#+ | 0.00 | 0.00+ | 0.00 | 0.00 | 0.00 | 0.1 1 | Mild |
| | 0.33 | | 0.00 | | 0.00 | | | |
| PVA | 0.33+ | 0.00 | 0.17+ | 0.00 | 0.00 | 0.00 | 0.1 7 | Mild |
| | 0.33 | | 0.17 | | 0.00 | | | |

As shown in Table 4, it has been found that irritation is significantly reduced in the microneedle sheet made of HPMC and the microneedle sheet made of PVA when compared with the microneedle sheet made of metal (SUS). It also has been confirmed that the microneedles are dissolved in skin in the case of the microneedle sheet made of HPMC and the microneedle sheet made of PVA. This experiment also indicates that the microneedle sheet including a soluble material as a material is less irritating to the skin and is highly safe.

The present invention has been described in detail above based on the embodiments and examples.

The applicator may have any shape and structure that is capable of bending the microneedle sheet 10 to raise the microneedles 12. For example, the applicator may be formed like a single straight rod. Alternatively, the applicator may include any mechanical, electrical, or electronic structure or control means. In connection with that the form of the applicator is not limiting, whether to use the liner 20 may be determined as desired.

The shape of the microneedle sheet is not limited to a strip but may be, for example, a rectangle having approximately equal length and width, or a circle or an oval. The applicator may not be used with some shapes of the microneedle sheet.

The microneedle sheet according to the present invention can be combined with electric (iontophoresis), pressure, magnetic-field, ultrasonic (sonophoresis), or other transdermal drug delivery techniques. A combination of the microneedle sheet with those other techniques can further increase the amount of drug absorption.

**Reference Signs List**

10 ... microneedle sheet, 11 ... principal surface, 12 ... microneedle, 20 ... liner, 30 ... applicator, 31 ... body, 32 ... first through hole, 33 ... second through hole, 34 ... adhesive.

**Claims**

**1.**  A microneedle sheet (10) comprising a plurality of microneedles (12) formed on a sheet generally along a principal surface (11) of the sheet, **characterized in that**

the microneedles (12) are formed of a material selected from among polyvinyl alcohol, and a graft copolymer of polyvinyl alcohol and polyethylene glycol,
the sheet has a thickness of 30 to 100 $\mu$m, and
the microneedles (12) are raised from the principal surface (11) by bending the sheet, and the raised microneedles (12) pierce skin.

2. The microneedle sheet (10) according to claim 1, wherein an insertion angle of the raised microneedle (12) to the skin is 34 degrees or greater and less than 180 degrees.

3. The microneedle sheet (10) according to claim 1 or 2, wherein a maximum angle between the microneedle (12) raised from the principal surface (11) and an imaginary line (V) extending from center of curvature of the sheet to a base of the microneedle (12) is greater than 90 degrees.

4. The microneedle sheet according to claim 3, wherein the maximum angle is 95 to 130 degrees.

**Patentansprüche**

1. Mikronadelfolie (10), umfassend eine Vielzahl von Mikronadeln (12), die auf einer Folie im Allgemeinen entlang einer Hauptoberfläche (11) der Folie gebildet sind, **dadurch gekennzeichnet, dass**

die Mikronadeln (12) aus einem Material gebildet sind, ausgewählt aus Polyvinylalkohol und einem Pfropfcopolymer aus Polyvinylalkohol und Polyethylenglykol,
die Folie eine Dicke von 30 bis 100 $\mu$m aufweist, und
die Mikronadeln (12) von der Hauptoberfläche (11) durch Biegen der Folie angehoben werden und die angehobenen Mikronadeln (12) die Haut durchstechen.

2. Mikronadelfolie (10) nach Anspruch 1, wobei ein Einführungswinkel der angehobenen Mikronadel (12) zur Haut 34 Grad oder mehr und weniger als 180 Grad beträgt.

3. Mikronadelfolie (10) nach Anspruch 1 oder 2, wobei ein maximaler Winkel zwischen der von der Hauptoberfläche (11) angehobenen Mikronadel (12) und einer imaginären Linie (V), die sich von der Mitte der Krümmung der Folie zu einer Basis der Mikronadel (12) erstreckt, größer als 90 Grad beträgt.

4. Mikronadelfolie nach Anspruch 3, wobei der maximale Winkel 95 bis 130 Grad beträgt.

**Revendications**

1. Feuille de micro-aiguilles (10) comprenant une pluralité de micro-aiguilles (12) formées sur une feuille généralement le long d'une surface principale (11) de la feuille, **caractérisée en ce que**

les micro-aiguilles (12) sont formées d'un matériau sélectionné parmi le poly(alcool de vinyle), et un copolymère greffé de poly(alcool de vinyle) et de poly(éthylène glycol),
la feuille ayant une épaisseur de 30 à 100 $\mu$m, et
les micro-aiguilles (12) étant élevées depuis la surface principale (11) par flexion de la feuille, et les micro-aiguilles (12) élevées perçant la peau.

2. Feuille de micro-aiguilles (10) selon la revendication 1, un angle d'insertion de la micro-aiguille (12) élevée vers la peau étant de 34 degrés ou plus et de moins de 180 degrés.

3. Feuille de micro-aiguilles (10) selon la revendication 1 ou 2, un angle maximal entre la micro-aiguille (12) élevée depuis la surface principale (11) et une ligne imaginaire (V) s'étendant depuis le centre de courbure de la feuille vers une base de la micro-aiguille (12) étant supérieur à 90 degrés.

4. Feuille de micro-aiguilles selon la revendication 3, l'angle maximal étant de 95 à 130 degrés.

## Fig.1

Fig.2

EP 3 111 987 B1

Fig.3

13

**Fig.4**

EP 3 111 987 B1

Fig.5

EP 3 111 987 B1

*Fig.6*

Fig.7

*Fig.8*

# Fig.9

| Classification | Material | Film formation | Properties | Laser processing | Bending resistance (mandrel test) | Raised angle of microneedle (°) (cylinder diameter 1.2 mm) | Insertion test | | Human skin (in vitro) |
|---|---|---|---|---|---|---|---|---|---|
| | | | | | | | Gel sheet | | Soluble state |
| | | | | | | | Insertion length (µm) (cylinder diameter 1.0 mm) | Insertion length (µm) (cylinder diameter 0.8 mm) | |
| Saccharides | Trehalose | × | Solution is repelled by the mold to form a mass | | | | | | |
| | Maltose | × | Solution is repelled by the mold to form a mass | | | | | | |
| | Hyaluronic acid | △ | Sheet becomes curved with drying | | | | | | |
| | Chondroitin sulfate sodium | △ | Sheet becomes curved with drying Cracked when folded | | | | | | |
| | Dextrin | × | Cracked in drying | | | | | | |
| | Pullulan | △ | Sheet becomes curved | | | | | | |
| | HPMC | ○ | | Possible | Resistant | 110 | 173 | 211 | Dissolved |
| | HPC | ○ | Low strength | Possible | - | - | - | - | - |
| Non-saccharides | Gelatin | △ | Polymer aggregates | | | | | | |
| | Polyacrylic acid | △ | | | | | | | |
| | Kollicoat IR | ○ | | Possible | - | - | - | - | - |
| | PVA | ○ | | Possible | Resistant | 114 | 173 | 201 | Dissolved |
| | PVP (K30) | △ | Sheet becomes cracked during drying | | | | | | |
| | PVP (K90) | △ | Sheet becomes curved with drying | | | | | | |

EP 3 111 987 B1

Fig.10

(a)

(b)

*Fig.11*

(a)

(b)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005503210 A
- WO 2013187392 A
- WO 2013191025 A
- EP 1360935 A
- US 20110034860 A